# EUROPÄISCHE PATENTSCHRIFT

(11) **EP 1 032 432 B1**
(45) Veröffentlichungstag und Bekanntmachung des Hinweises auf die Patenterteilung: **15.01.2003**
(21) Anmeldenummer: 98965164.1
(22) Anmeldetag: 18.11.1998
(51) Int. Cl.: A61L 2/00

(54) **DAMPFSTERILISATIONS- BZW. DAMPFDESINFEKTIONSVERFAHREN UND VORRICHTUNG ZUR DURCHFÜHRUNG DES VERFAHRENS**
STEAM STERILISATION OR STEAM DISINFECTING METHOD AND DEVICE FOR CARRYING OUT THE METHOD
PROCEDE DE STERILISATION A LA VAPEUR ET DE DESINFECTION A LA VAPEUR ET DISPOSITIF POUR METTRE EN OEUVRE LEDIT PROCEDE

(30) Priorität: 21.11.1997 DE 19751692
(43) Veröffentlichungstag der Anmeldung: 06.09.2000
(73) Patentinhaber: MMM MÜNCHENER MEDIZIN MECHANIK GMBH, D-81369 München (DE)
(72) Erfinder: ACHTERBERG, Dieter, D-86949 Windach (DE); GODER, Franz, D-81375 München (DE)
(74) Vertreter: Grünecker, Kinkeldey, Stockmair & Schwanhäusser Anwaltssozietät
(86) Internationale Anmeldenummer: EP9807394
(87) Internationale Veröffentlichungsnummer: WO99026667

(56) Entgegenhaltungen:
- EP-A- 0 848 958
- WO-A-95/14494
- DE-A- 3 340 050
- US-A- 4 203 947
- US-A- 4 238 447
- US-A- 4 335 071

## Beschreibung

Die Erfindung betrifft ein Dampfsterilisations- bzw. Dampfdesinfektionsverfahren zur Behandlung von Sterilgut im Inneren einer Kammer gemäß dem Oberbegriff des Patentanspruchs 1. Die Erfindung betrifft ferner eine Vorrichtung zur Durchführung eines Dampfsterilisations- bzw. Desinfektionsverfahrens von Sterilgut im Innern einer Kammer gemäß dem Oberbegriff des Patentanspruchs 21.

Aus der US 4,238,447 ist ein derartiges Dampfsterilisations- bzw. Desinfektionsverfahren bekannt, wobei dort im Rahmen eines vorbereitenden Schrittes vor dem Sterilisationsvorgang erst ein Vakuum im Innern der Kammer erzeugt und anschließend Dampf eingebracht wird. Das in der Kammer aus darin verbliebenem Gasmedium und eingebrachten Dampf gebildete Mischmedium wird nach einer vorher festgelegten Zeitspanne mittels einer Pumpe herausgepumpt. Als eine solche Pumpe wird eine Saugstrahlpumpe oder eine Seitenkanalpumpe vorgeschlagen.

Ein derartiges Dampfsterilisations- bzw. Desinfektionsverfahren ist ferner im einzelnen beschrieben in DIN 58 946 bzw. CEN 285. Diese Dampfsterilisations- bzw. Desinfektionsverfahren lassen sich in die Hauptschritte Luftentfernung, Sterilisieren und Trocknen untergliedern.

Im Rahmen des Verfahrensschrittes "Luftentfernung" wird die bei der Beschickung der Kammer mit dem Sterilgut in die Kammer gelangte Luft aus dieser entfernt.

Im Rahmen des anschließend durchgeführten Verfahrensschrittes "Sterilisieren" wird Sattdampf unter Aufrechterhaltung eines bestimmten Dampfzustandes mit dem Sterilgut für eine vorbestimmte Einwirkzeit in Kontakt gebracht zum vollständigen Abtöten etwaiger, dem Sterilgut anhaftender Keime.

Im Rahmen des nachfolgend durchgeführten Verfahrensschrittes "Trocknen" wird das Sterilgut getrocknet und abschließend die Kammer belüftet, so daß die Kammer zum Entnehmen des Sterilgutes geöffnet werden kann.

Wenigstens im Rahmen des Verfahrensschrittes "Trocknen" und üblicherweise auch im Rahmen des Verfahrensschrittes "Luftentfernung" wird der Druck im Inneren der Kammer für eine vorgegebene Zeitperiode unter den Umgebungsdruck mittels einer Wasserring-Vakuumpumpe abgesenkt. Die Wasserring-Vakuumpumpe ist hierzu an eine Kühlwasser-Quelle angeschlossen. Die Kühlwasser-Temperatur bestimmt den bei der Luftentfernung sowie beim Trocknen jeweils erreichbaren Unterdruck-Pegel.

Die bislang angewandten Dampfsterilisations- bzw. Desinfektionsverfahren haben sich insbesondere im Hinblick darauf, daß diese auch bei Verzicht auf toxische Sterilisiermittel eine vergleichsweise hohe Sterilisations- bzw. Desinfektionswirkung haben, als vorteilhaft erwiesen. Die wesentlichen Betriebskosten einer zur Durchführung eines Dampfsterilisations- bzw. Desinfektionsverfahren vorgesehenen Anlage werden durch den Kühlwasserverbrauch, den Dampfverbrauch und die Energie für den Betrieb der Vakuumpumpe verursacht. Das Kühlwasser dient zur Kondensation des Dampfes und als Betriebsmittel für die Wasserring-Vakuumpumpe.

Der Erfindung liegt die Aufgabe zugrunde, die Durchführung eines Dampfsterilisations- bzw. Desinfektionsverfahrens zur Behandlung von Sterilgut im Inneren einer Kammer unter verringerten Betriebskosten zu ermöglichen.

Diese Aufgabe wird erfindunsgemäß durch ein Dampsterilisationsverfahren mit den in Patentanspruch 1 angegebenen Merkmalen gelöst.

Dadurch wird es auf vorteilhafte Weise möglich, insbesondere den im Rahmen des Trocknungsschrittes anfallenden Kühlwasserverbrauch erheblich zu verringern und den Trocknungsschritt ggf. gänzlich ohne Kühlwasser durchzuführen. Ferner wird es auf vorteilhafte Weise möglich, anstelle der bislang verwendeten, im Betrieb vergleichsweise teuren Wasserringmantel-Vakuumpumpen eine ungekühlte Pumpe, insbesondere eine trocken laufende Drehkolbenpumpe, zu verwenden.

Weiterhin wird es auf vorteilhafte Weise möglich, im Rahmen des vorbereitenden, der Luftentfernung dienenden Verfahrensschrittes die Luftentfernung unter lediglich kurzzeitigem Betrieb mit einer ggf. ungekühlten Vakuumpumpe vorzunehmen. Durch die anfangs vorgenommene Absenkung des Druckes in der Kammer auf einen vergleichsweise niedrigen Druckpegel von beispielsweise 20 mbar und die anschließende Zufuhr von Dampf in die Kammer auf einen Überdruck von beispielsweise 450 mbar und die nachfolgende Entspannung des in der Kammer befindlichen Mediums auf Umgebungsdruck wird die beim Einbringen des Sterilguts in die Sterilisierkammer gelangte Luft bereits weitestgehend aus der Kammer entfernt.

In weiterhin besonders vorteilhafter Weise wird es möglich, die Trocknung des behandelten Sterilisiergutes zu beschleunigen.

Bei dem zur Verdrängung des Sterilisierdampfes in die Kammer in vorteilhafter Weise von unten großflächig eingeführten Gasmedium handelt es sich vorzugsweise um gefilterte, keimfreie und getrocknete Luft. Alternativ dazu ist es auch möglich, das Verdrängermedium aus einer Stickstoff/Kohlendioxyd-Mischung oder anderen Gasen zu bilden.

Um eine möglichst effektive Verdrängung des Sterilisierdampfes zu erreichen, wird in vorteilhafter Weise das im Rahmen des Trocknungsschrittes eingebrachte Gasmedium derart in die Kammer eingebracht, daß dieses den in der Kammer noch befindlichen Dampf zu einem Ablaßabschnitt hin verdrängt. Dadurch wird auf vorteilhafte Weise eine Vermengung des eingebrachten Gas-Mediums, insbesondere der gefilterten Luft, mit dem zu verdrängenden Dampf weitgehend vermieden.

Um eine möglichst vollständige Verdrängung des Dampfes durch das im Rahmen des Trocknungsschrittes der Kammer zugeführte Gas-Mediums zu erreichen, wird in vorteilhafter Weise im Rahmen des Trocknungsschrittes das Gas-Medium solange zugeführt, bis in der Kammer ein Druck herrscht, der höher ist als der Umgebungsdruck. Das hierbei in der Kammer gebildete, teils aus dem zugeführten Gas-Medium und teils aus Resten des Sterilisier-Dampfes bestehende Medium kann dabei zunächst ohne Zuhilfenahme einer Fördereinrichtung aufgrund des im Inneren der Kammer herrschenden, ggf. nur geringen Überdruckes abgeblasen werden.

Insbesondere in der Anfangsphase des Trocknungsschrittes wird der abgeblasene Sterilisierdampf in vorteilhafter Weise über eine Wärmetauschereinrichtung geleitet, durch die das der Sterilisierkammer vorzugsweise im unteren Bereich zugeführte Gas-Medium erwärmt wird, zur Steigerung der Trocknungswirkung. Dadurch wird auf vorteilhafte Weise zumindest ein Teil der in dem abgeblasenen Sterilisierdampf enthaltenen Wärmemenge zur Unterstützung des Trocknungsvorganges zurückgewonnen.

Um zumindest in der Anfangsphase des Trocknungsschrittes eine weitgehende Durchmischung des Sterilisierdampfes mit dem zur Verdrängung des Sterilisierdampfes in die Kammer eingebrachten Gas-Mediums zu verhindern, wird das Gas-Medium in vorteilhafter Weise unter Bildung einer Verdrängungsströmung in die Kammer eingeführt. Diese Verdrängungsströmung ist dabei in vorteilhafter Weise derart gerichtet, daß die Verdrängung des Sterilisierdampfes in vertikaler Richtung von unten nach oben erfolgt, Die Ausbildung der Verdrängungsströmung wird in vorteilhafter Weise durch eine Lochblechanordnung und ggf. eine weitere Zwischenlage unterstützt, so daß das zur Verdrängung vorgesehene Gas-Medium großflächig in die Kammer eintritt. Auch der Abzug des verdrängten Sterilisierdampfes erfolgt in vorteilhafter Weise über einen großflächig ausgebildeten Ablaßabschnitt, der in gleicher Weise wie der Einlaßbereich mit einer Lochblechanordnung versehen sein kann. Der Ablaßabschnitt ist in vorteilhafter Weise im oberen Bereich der Kammer ausgebildet. In vorteilhafter Weise wird die Zuströmgeschwindigkeit in die Kammer dabei auf einen vergleichsweise geringen Wert, beispielsweise 0,1 m/sek eingestellt.

Eine besonders vorteilhafte Ausförderung des nach Abschluß des Sterilisierschrittes in der Kammer befindlichen Sterilisierdampfes wird dadurch erreicht, daß das zur Verdrängung des Sterilisierdampfes vorgesehene Gas-Medium in einen unteren Bereich der Kammer eingeleitet wird und in einem oberen Bereich der Kammer abgeleitet wird. Durch entsprechend großflächige Ausgestaltung des Eintritts- und des Austsrittsabschnittes kann dadurch erreicht werden, daß der in der Kammer befindliche Sterilisierdampf vor einer durch das nachströmende Gas-Medium gebildeten Gas-Front vorgeschoben und zu dem Austrittsbereich hin gedrängt wird.

Vorteilhafte Weiterbildungen dieses Verfahrens sind Gegenstand der Unteransprüche.

Hinsichtlich einer Vorrichtung wird die eingangs angegebene, der Erfindung zugrundeliegende Aufgabe durch eine Vorrichtung mit den in Patentanspruch 21 angegebenen Merkmalen gelöst.

Die Steuereinheit ist in vorteilhafter Weise durch eine elektronische Steuereinrichtung gebildet, die entsprechende Verfahrensabläufe auswählt und die Ventileinrichtungen zeitlich derart steuert, daß im Rahmen des Trocknungsschrittes das nicht kondensierbare Gas, insbesondere die Luft vor Absenken des Druckes in die Kammer einströmen kann und der dadurch verdrängte Dampf über eine Ableitungseinrichtung aus der Kammer austreten kann. Hierdurch wird erreicht, daß beim Absenken des Druckes in der Kammer zur Trocknung des Sterilgutes die entsprechende Vakuumpumpe nicht Wasserdampf sondern lediglich Luft fördert. Dadurch wird es möglich, auf eine Kühlung der Pumpe zu verzichten.

Durch die erfindungsgemäße Vorrichtung wird es auf apparatetechnisch vorteilhafte Weise möglich, ein Dampfsterilisations- bzw. Dampfdesinfektionsverfahrens ohne Kühlwasser durchzuführen.

Vorteilhafte Weiterbildungen dieser Vorrichtung sind in den Unteransprüchen angegeben.

Weitere Einzelheiten und Merkmale der vorliegenden Erfindung ergeben sich aus der nachfolgenden Beschreibung einer bevorzugten Ausführungsform eines Systems zur Durchführung des erfindungsgemäßen Dampfsterilisations- bzw. Desinfektionsverfahrens in Verbindung mit den Zeichnungen. Es zeigen:
- Fig.1: eine Schemadarstellung eines Systems zur Durchführung des Dampfsterilisations- bzw. Dampfdesinfektionsverfahrens,
- Fig.2: eine Schemadarstellung eines Steuerungsablaufes zur Durchführung des Sterilisations- bzw. Desinfektionsverfahrens.

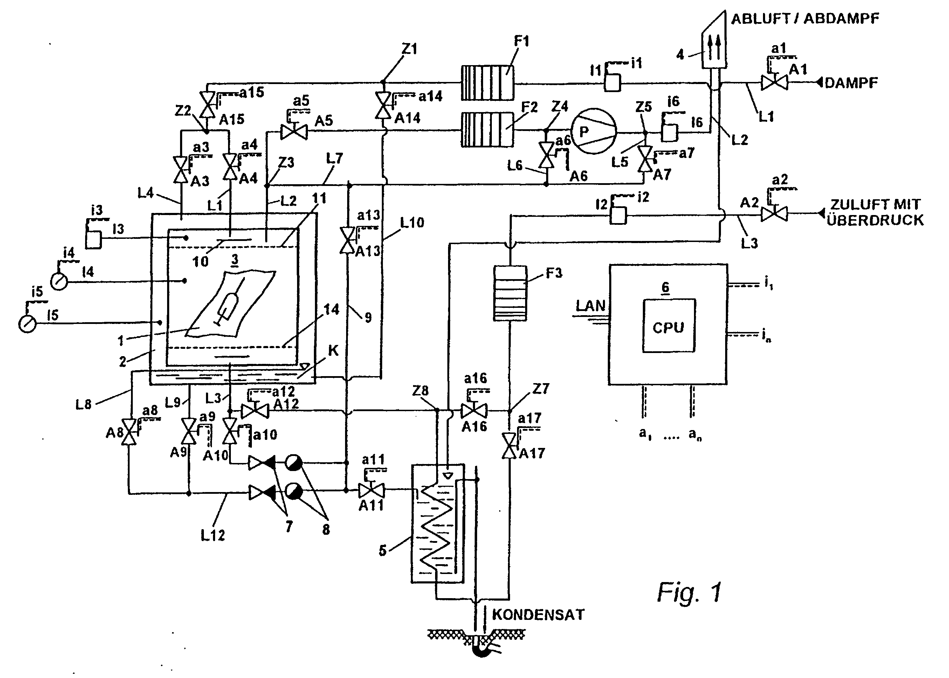

Das in Fig.1 dargestellte System zur Durchführung des erfindungsgemäßen Dampfsterilisations- bzw. Dampfdesinfektionsverfahrens umfaßt eine zur Aufnahme von zunächst unbehandeltem Sterilgut 1 vorgesehene, von einem Mantel 2 umgebene Kammer 3. Der Innenraum der Kammer 3 steht über eine erste Leitungseinrichtung L1 mit einer Dampfquelle in Verbindung.

In der ersten Leitungseinrichtung L1 sind bei der hier dargestellten Ausführungsform ein erstes Ventil A1, ein Dampf-Zustandssensor I1, ein Dampffilter F1 zur Filterung des Dampfes hinsichtlich Schmutz und/oder Pyrogene, ein Verzweigungsstück Z1, ein weiteres, automatisch steuerbares Ventil A15 und ein Kesselventil A4 angeordnet. Die Ventile A1, A15 und A4 sind nach Maßgabe einer elektronischen Steuereinrichtung 6 steuerbar. Die Ventile A1, A15 und A4 sind als Servoventile ausgebildet und werden über entsprechende Signale a1, a15, a4 angesteuert.

In der ersten Leitungseinrichtung L1 ist eine weitere Verzweigungsstelle Z2 vorgesehen, über welche der Mantel 2 mit Dampf beaufschlagbar ist. In einer, an der Verzweigungsstelle Z2 von der ersten Leitungseinrichtung L1 abzweigenden Leitungseinrichtung L4 ist ein ebenfalls nach Maßgabe der elektronischen Steuereinrichtung 6 steuerbares Ventil A3 vorgesehen.

Die Leitungseinrichtung L1 mündet bei der hier dargestellten Ausführungsform in einen oberen Bereich der Kammer 3. Der Mündungsbereich der ersten Leitungseinrichtung L1 ist von einem Prallblech 10 überdeckt. Der obere Bereich der Kammer 3 ist ferner nahezu vollflächig von einem Lochblech 11 verkleidet. Dadurch wird ein gleichmäßiges Zuströmen des über die erste Leitungseinrichtung L1 zugeführten Dampfes erreicht.

Die Kammer 3 steht über eine zweite Leitungseinrichtung L2 mit einem Abluft- bzw. einem Abdampfkanal 4 in Verbindung. In der zweiten Leitungseinrichtung L2 sind ein nach Maßgabe der elektronischen Steuereinrichtung schaltbares Ventil A5, ein Abluft/Abdampffilter F2, eine hier als trocken laufende Drehkolbenpumpe ausgebildete Vakuumpumpe P sowie ein Abluft/Abdampfsensor 16 vorgesehen.

In der zweiten Leitungseinrichtung L2 sind mehrere Abzweigstellen Z3, Z4 und Z5 vorgesehen. An diesen Abzwelgstellen zweigen von der zweiten Leitungseinrichtung L2 mehrere Abzweigleitungen L5, L6 und L7 ab. Die Vakuumpumpe P ist in der zweiten Leitungseinrichtung L2 zwischen zwei Abzweigstellen Z4, Z5 angeordnet. Durch entsprechende Ansteuerung von Ventilen A6, A7 mittels der elektronischen Steuereinrichtung 6 ist es möglich, die Vakuumpumpe P über die Leitungseinrichtung L5 zu umgehen. Durch Schließen des Ventiles A5 und Öffnen des Ventiles A6 oder A7 ist es ferner möglich, den Abluft/Abdampffilter F2 zu umgehen.

Die an der Abzweigstelle Z3 von der zweiten Leitungseinrichtung L2 abzweigende Abzweigleitung L7 ist über eine weitere Ventileinrichtung A13 und über ein nach Maßgabe der elektronischen Steuereinrichtung 6 kontinuierlich aufsteuerbares Wärmetauscherheizventil A11 mit einem Wärmetauscher 5 verbindbar.

Der Wärmetauscher 5 ermöglicht eine weitgehende Rückgewinnung der Wärmemenge eines, insbesondere über die zweite Leitungseinrichtung L2 aus der Kammer 3 entweichenden Mediums. Der Wärmetauscher 5 ist dazu vorgesehen, ein über eine dritte Leitungseinrichtung L3 der Kammer 3 zugeführtes Gasmedium, insbesondere Luft, aufzuwärmen. Der Wärmetauscher 5 ist hierbei über zwei Abzweigstellen Z7, Z8 an die dritte Leitungseinrichtung L3 angeschlossen. Die dritte Leitungseinrichtung umfaßt ein nach Maßgabe der elektronischen Steuereinheit 6 steuerbares Ventil A12, ein zwischen den Abzweigstellen Z7 und Z8 angeordnetes Schaltventil A16, einen Zuluftfilter F3 zum Filtern der über die dritte Leitungseinrichtung L3 dem Kessel 3 zugeführten Zuluft, sowie einen Zuluftsensor 12 und ein Zuluftschaltventil A2. Die Ventile A16 und A2 sowie der Zuluftsensor 12 sind ebenfalls an die elektronische Steuereinrichtung 6 angeschlossen.

Der die Kammer 3 umgebende Mantel 2 steht neben der Leitungseinrichtung L4 mit einer Kondensatpegelleitung L8, einer Kondensatableitungsleitung L9 und einer von der ersten Leitungseinrichtung L1 abzweigenden Dampfzufuhrleitung L10 in Verbindung.

Die Dampfzufuhrleitung L10 ist schaltbar über eine Ventileinrichtung A14 mit der ersten Leitungseinrichtung L1 verbindbar.

Über die Dampfzufuhrleitung L10 ist es möglich, Dampf von der ersten Leitungseinrichtung L1 abzuzweigen und diesen in den Mantel 2 derart einzuleiten, daß der Dampf durch das im Mantel 2 befindliche Kondensat ggf. befeuchtet oder gekühlt wird. Hierzu mündet die Dampfzufuhrleitung L10 unmittelbar in ein im Bodenbereich des Mantels 2 gebildetes Kondensatauffangbecken. Der durch das Kondensatauffangbekken hindurchperlende Dampf kann durch entsprechende Ansteuerung der Ventile A3 und A4 über die Leitungseinrichtungen L4 und L1 in die Kammer 3 eingebracht werden. Dadurch ist auf vorteilhafte Weise sichergestellt, daß der der Kammer zugeführte Dampf nicht überhitzt ist.

Zur Überwachung des Zustandes des der Kammer 3 zugeführten Dampfes ist ein Dampfzustandssensor 13 vorgesehen, der bei der hier dargestellten Ausführungsform noch in dem von der Kammer 3 über das Lochblech 11 abgetrennten Raumabschnitt angeordnet ist. Der Dampfzustandssensor 13 ist ebenfalls an die elektronische Steuereinrichtung 6 angeschlossen. Zur Überwachung des Druckes im Inneren der Kammer 3 ist ein an die elektronische Steuereinrichtung 6 angeschlossener Kammerdrucksensor 14 vorgesehen.

Der Druck im Inneren des Mantels 2 wird von einem ebenfalls an die elektronische Steuereinrichtung 6 angeschlossenen Manteldrucksensor 15 überwacht.

Das ggf. aus dem Mantel 2 abgezogene Kondensat kann ebenfalls dem Wärmetauscher 5 zu Wärmetauschzwecken, insbesondere zum Aufheizen der im Rahmen eines Trocknungsvorganges der Kammer 3 zugeführten Luft, verwendet werden. Hierzu ist eine Kondensatablaufleitung L12 vorgesehen, die mit der Kondensatpegelleitung L8 und der Kondensatableitungsleitung L9 in Verbindung steht und mit einem Rückschlagventil 7 und einem Kondensatableiter 8 versehen ist. Über ein nach Maßgabe der elektronischen Steuereinrichtung 6 steuerbares Kessel-Bodenventil A10 ist es möglich, etwaiges, in der Kammer 3 gebildetes Kondensat aus der Kammer 3 vor Durchführung eines Trockenvorganges abzuleiten. Das abgeleitete Kondensat kann ebenfalls über ein Rückschlagventil 7 und einen Kondensatableiter 8 dem Wärmetauscher 5 zugeführt werden.

Sobald das gesamte, in der Kammer 3 angesammelte Kondensat über das Kesselbodenventil A10 aus der Kammer 3 abgeleitet ist, kann durch Öffnen eines in einer Abzweigleitung 9 angeordneten Ventiles A13 Dampf aus der Kammer 3 abgezogen und in den Abluft/Abdampfkanal 4 abgelassen werden.

Die elektronische Steuereinrichtung 6 ist derart ausgebildet, daß diese die für die Dampfzufuhr, die Druckluftzufuhr und die Abluft/Abdampfableitungen L1, L3 und L2 maßgeblichen Ventile derart ansteuert, daß die im Rahmen eines Trocknungsvorganges der Kammer 3 zugeführte Zuluft bedarfsweise vorab aufgeheizt werden kann. Die zeitliche Abfolge der Ansteuerung der Ventile erfolgt derart, daß vor Absenken des Druckes im Inneren der Kammer im Rahmen des Trocknungsschrittes der überwiegende Teil des nach dem Sterilisierschritt noch in der Kammer befindlichen Dampfes vorab weitgehend aus der Kammer 3 verdrängt wird. Aufgrund des extrem verringerten Wassergehaltes im Inneren der Kammer 3 kann das zur Trocknung des Sterilgutes 1 erforderliche Hochvakuum durch die Pumpe P erzeugt werden, ohne daß hierbei die Pumpe P in besonderem Maße gekühlt werden muß.

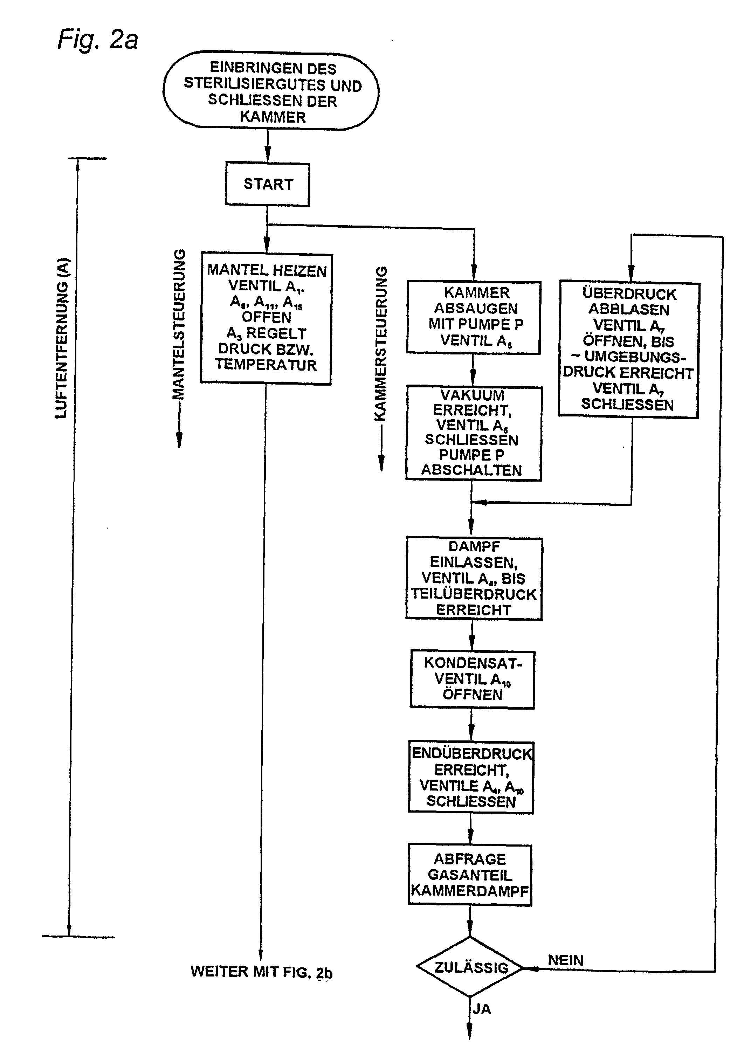

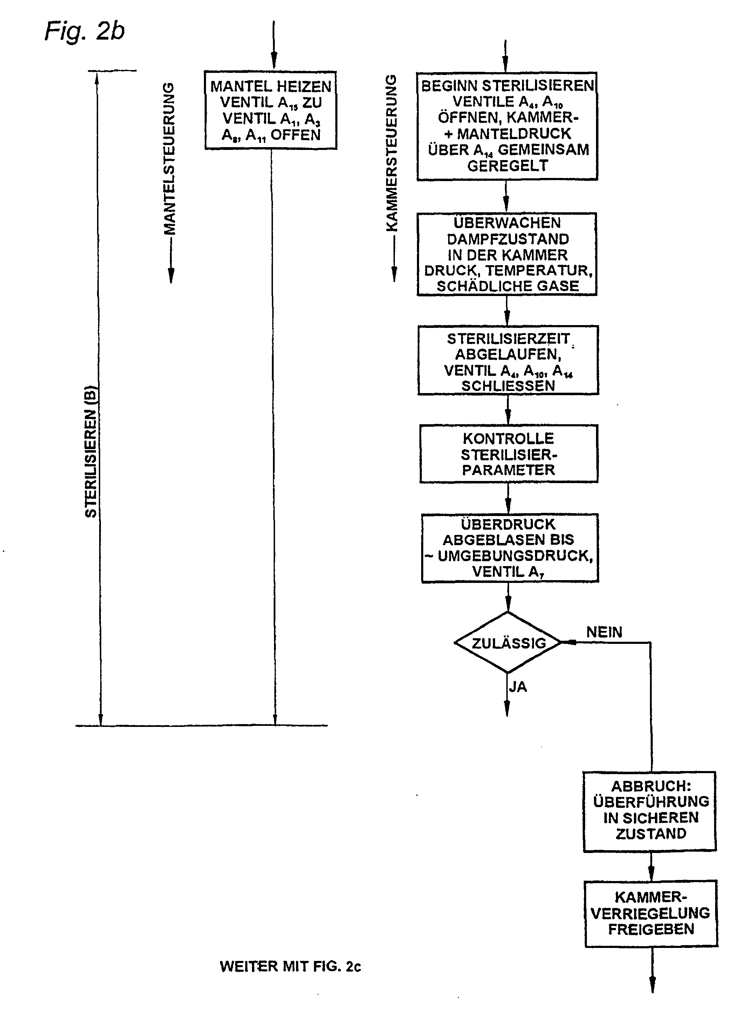

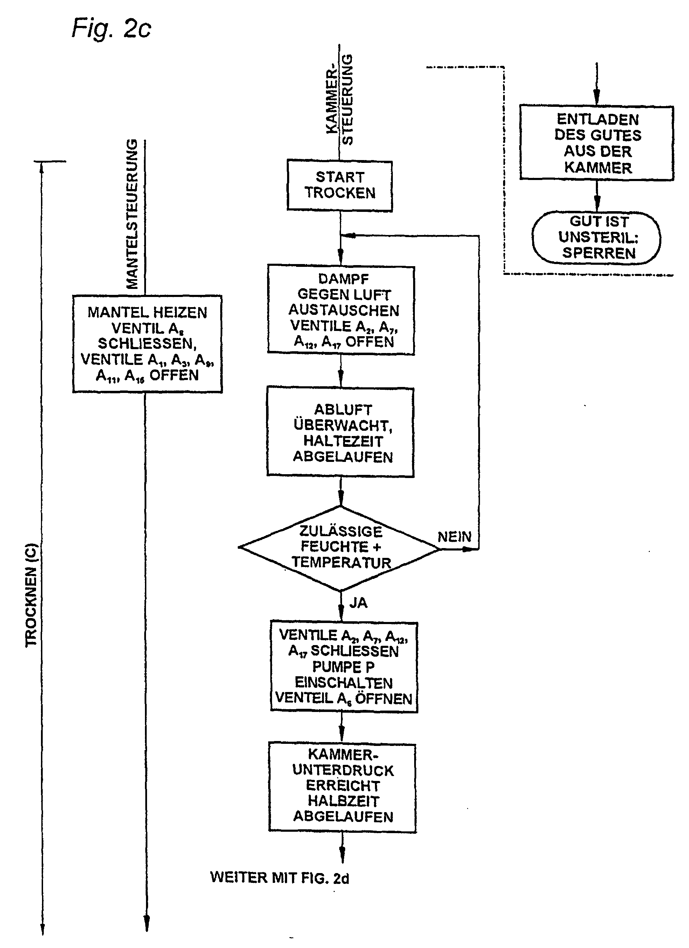

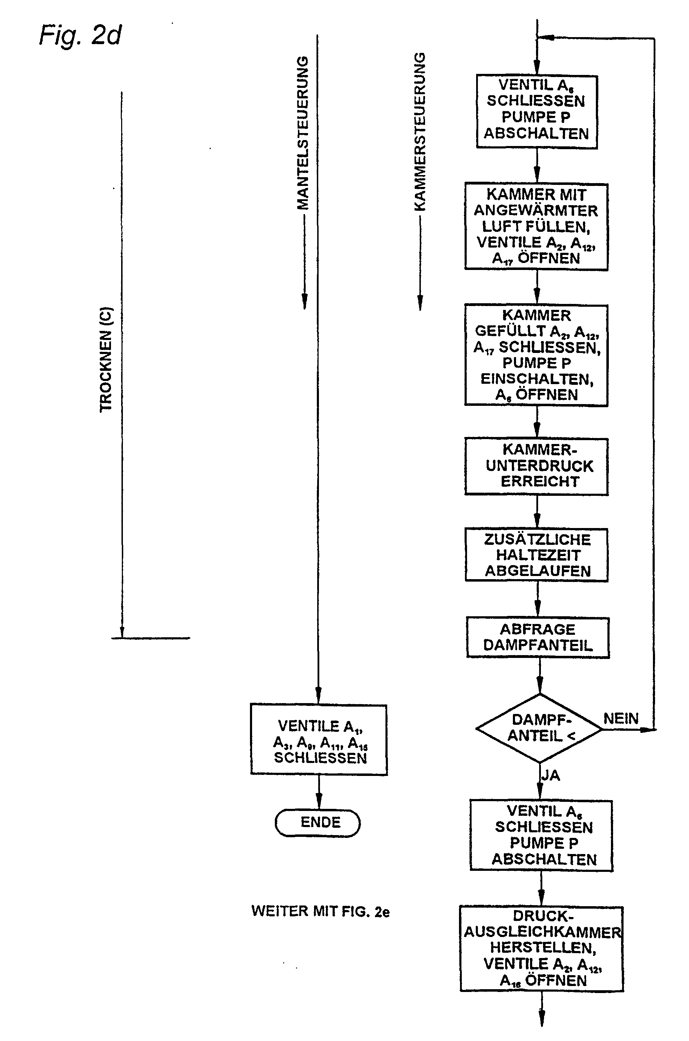

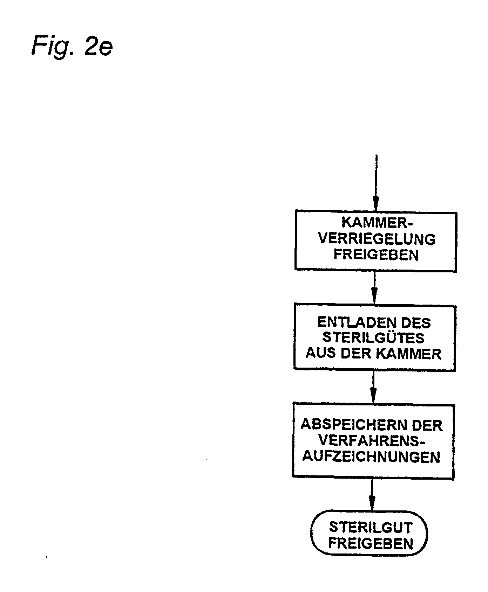

Die Funktionsweise des in Fig.1 dargestellten Systems wird nachfolgend unter Bezugnahme auf das in den Fig.2a bis 2e dargestellte Flußdiagramm beschrieben.

### Vorbereitung (A)

Zur Behandlung von Sterilgut im Rahmen eines Dampfsterilisationsverfahrens wird dieses in eine Sterilisierkammer 3 eingebracht. Hierzu wird die Kammer 3 geöffnet und das Sterilgut in der Kammer 3 abgelegt. Die Kammer 3 wird verschlossen. Entsprechend eines in einer elektronischen Steuereinrichtung 6 abgespeicherten Arbeitsverfahrens wird der Mantel 2 der Sterilisierkammer 3 beheizt. Hierzu wird das Ventil A1 durch einen von der elektronischen Steuereinrichtung 6 ausgegebenen Steuerbefehl a1 angesteuert und geöffnet. Der von einem hausseitigen Dampfnetz bereitgestellte Dampf, insbesondere Sattdampf durchströmt die erste Leitungseinrichtung L1, den Filter F1, das ebenfalls nach Maßgabe der Steuereinheit 6 geöffnete Ventil A15 und das entsprechend einem Steuerbefehl a3 durch die Steuereinrichtung 6 geöffnete Ventil A3. Der Druck im Inneren des Mantels 2 wird durch den Drucksensor 15 überwacht. Ein durch den Drucksensor 15 erzeugter Meßwert i5 wird hierbei der elektronischen Steuereinrichtung 6 zugeführt. Der Druck und die Temperatur im Inneren des Mantels 2 werden durch entsprechende Ansteuerung des Ventiles A3 sowie der Ventile A8 und A9 geregelt. Das sich im Bodenbereich des Mantels 2 bildende Kondensat wird über die Kondensatpegelleitung L8 sowie über die Kondensatableitungsleitung L9 aus dem Mantel 2 abgeleitet. Hierzu werden die Ventile A8 und A9 durch entsprechend von der Steuereinrichtung 6 ausgegebene Steuerbefehle a8, a9 betätigt.

Parallel zum Aufheizen des Mantels 2 der Sterilisierkammer 3 wird aus der Sterilisierkammer 3 die beim Einbringen des Sterilgutes 1 in die Kammer gelangte Luft aus dieser entfernt. Hierzu wird das Ventil A5 entsprechend einem von der Steuereinrichtung 6 ausgegebenen Steuerbefehl a5 geöffnet und die als trocken laufende Drehkolbenpumpe ausgebildete Vakuumpumpe P in Gang gesetzt. Die hierbei über die Leitungseinrichtung L2 von der Pumpe angesaugte Luft wird vor Eintritt in die Pumpe durch den Filter F2 gefiltert. Der Druck im Inneren der Kammer wird auf einen extrem niedrigen Druckpegel, beispielsweise 20 mbar, abgesenkt. Anschließend wird das Ventil A5 geschlossen und die Pumpe P abgeschaltet.

Nunmehr wird das in der ersten Leitungseinrichtung L1 angeordnete Ventil A4 geöffnet und über die Leitungseinrichtung L1 der Sterilisierkammer 3 solange Dampf zugeführt, bis im Inneren der Sterilisierkammer 3 gegenüber dem Umgebungsdruck ein Teil-Überdruck von beispielsweise 450 mbar herrscht.

Anschließend wird entsprechend einem von der elektronischen Steuereinheit ausgegebenen Befehl a10 das Ventil A10 geöffnet und das in der Sterilisierkammer 3 gebildete Misch-Medium aus Dampf und Rest-Luft über die Leitungseinrichtung L3 aus der Sterilisierkammer abgelassen. Aufgrund des in der Sterilisierkammer 3 zunächst herrschenden Überdrucks entströmt das in der Kammer 3 gebildete Misch-Medium aus Restluft und Dampf der Kammer 3, ohne daß es hierzu einer Pumpe bedarf.

Sobald der Druck im Inneren der Sterilisierkammer 3 wieder auf den Umgebungsdruck abgefallen ist, wird das Ventil A10 geschlossen. Auch das Ventil A4 wird spätestens jetzt entsprechend einem von der Steuereinrichtung 6 ausgegebenen Steuerbefehl a4 geschlossen.

Vermittels des Dampfzustandsensors 13 wird der Gas- bzw. Luftanteil in dem in der Kammer 3 befindlichen Medium abgefragt. Falls der Anteil an nicht kondensierbaren Gasen in dem in der Kammer 3 befindlichen Dampf einen bestimmten Grenzwert überschreitet, wird nochmals das Ventil A4 geöffnet, bis erneut der vorgegebene Teildruckpegel von ca. 450 mbar erreicht wird. Anschließend wird erneut durch Öffnen des Ventiles A10 über die Leitungseinrichtung L3 das in der Sterilisierkammer befindliche Medium auf Umgebungsdruck entspannt. Unter Berücksichtigung des von dem Dampfzustandssensor 13 erfaßten Maßsignales i3 steuert die Steuereinrichtung 6 die Ventile A4 und A10 solange wechselweise in eine Offen- bzw. Schließstellung, bis der von dem Dampfzustandssensor 13 ermittelte Restgehalt an nicht kondensierbaren Gasen unter einem vorgegebenen Grenzwert liegt. Sobald der Restluftgehalt im Inneren der Sterilisierkammer 3 unter dem vorgegebenen, maximal zulässigen Grenzwert liegt, kann mit dem zur vollständigen Abtötung der dem Sterilgut anhaftenden Keime führenden Sterilisierschritt begonnen werden.

### Sterilisierschritt (B)

Nachdem im Rahmen des vorbereitenden Verfahrensschrittes die Menge an nicht kondensierbaren Gasen im Inneren der Sterilisierkammer 3 unter einen vorgegebenen Grenzwert abgesenkt wurde, wird das Ventil A4 geöffnet und über die Leitungseinrichtung L1 gefilterter Dampf der Sterilisierkammer 3 innerhalb einer bestimmten Steigzeit zugeführt, bis ein bestimmter Sterilisier-Überdruckpegel von beispielsweise 1050 mbar erreicht ist. Durch eine über die Steuereinrichtung 6 gesteuerte Ansteuerung der Ventile A4 und A10 sowie ggf. durch weiteres Aufheizen des Mantels 2 wird im Inneren der Sterilisierkammer ein hinsichtlich Temperatur und Druck und ggf. auch Dampfnässe definierter Dampfzustand, beispeilsweise T = 121°C, p_{abs} = 2,0490 bar, über eine bestimmte Haltezeit von beispielsweise 12 Minuten hinweg aufrechterhalten. Der Druck, die Temperatur bzw. der Dampfzustand des in der Kammer 3 befindlichen Dampfes werden weiterhin überwacht und die entsprechenden Meßsignale i4, i3 der Steuereinrichtung 6 zugeführt.

Nachdem die vorbestimmte Sterilisierzeit abgelaufen ist, werden die Ventile A4 und, sofern das Ventil A10 ggf. leicht geöffnet war, auch dieses geschlossen. Die für den Sterilisierschritt charakteristischen Parameter werden von der Steuereinrichtung 6 überprüft und aufgezeichnet.

Um zu vermeiden, daß das Sterilgut 1 im Rahmen des Sterilisierschrittes mit überhitztem Dampf in Kontakt gelangt, ist es möglich, den der Sterilisierkammer 3 zugeführten Dampf nicht unmittelbar der Leitungseinrichtung L1 zu entnehmen und in die Sterilisierkammer 3 einzuführen, sondern den Sterilisierdampf zunächst in den Mantel 2, insbesondere in das in dem Mantel 2 befindliche Kondensat K einzuleiten und durch Öffnen der Ventile A3 und A4 über die Leitungseinrichtung L4 aus dem oberen Bereich des Mantels 2 den unter Druck stehenden Dampf abzuleiten und in die Sterilisierkammer 3 zu führen. Da der über die Leitungseinrichtung L10 zunächst dem Mantel 2 zugeführte Dampf mit dem Kondensat K in Kontakt gelangt, wird auf zuverlässige Weise gewährleistet, daß der in die Sterilisierkammer 3 eintretende Dampf nicht überhitzt ist.

Zur Beendigung des Sterilisierschrittes werden die Ventile A3 und A4 geschlossen und das Kondensatventil A10 geöffnet, bis der Druck im Inneren der Kammer 3 auf Umgebungsdruck entspannt ist. Der hierbei aus der Sterilisierkammer 3 abströmende Dampf wird über das durch die Steuereinrichtung 6 gesteuerte Ventil A11 dem Wärmetauscher 5 zugeführt. Das sich in dem Wärmetauscher 5 ggf. bildende überschüssige Kondensat wird über eine Ablaufeinrichtung abgeführt.

### Trocknungsschritt (C)

Das nunmehr durch den vorangehend beschriebenen Sterilisierschritt sterilisierte Sterilgut 1 wird im Rahmen des folgenden Trocknungsschrittes getrocknet. Hierzu wird der Mantel 2 weiter geheizt, in dem der Öffnungsgrad der Ventile A1, A3, A9 und A15 nach Maßgabe der Steuereinrichtung 6 durch Befehle a₁, a₃, a₉ und a₁₅ gesteuert wird.

Um das in der Sterilisierkammer 3 befindliche, auf Umgebungsdruck entspannte Dampfmedium rasch aus der Sterilisierkammer 3 zu entfernen, wird vor Inbetriebnahme der Pumpe zunächst über die Leitungseinrichtung L3 Druckluft der Sterilisierkammer 3 zugeführt. Hierzu wird das Ventil A2 geöffnet und die der Sterilisierkammer 3 zuströmende Druckluft durch einen Zuluftfilter F3 gegen Rekontamination des Sterilguts 1 gefiltert. Durch entsprechende Ansteuerung der Ventile A16 und A17 kann die gefilterte Druckluft bedarfsweise durch den Wärmetauscher 5 geleitet und dabei erwärmt werden. Durch den Öffnungsgrad der Ventile A16 und A17 läßt sich auch bei einem vergleichsweise stark aufgeheizten Wärmetauscher die maximal zulässige Temperatur der zugeführten Druckluft präzise steuern. Der Zustrom der ggf. durch den Wärmetauscher 5 aufgeheizten Druckluft wird ferner auch durch das ebenfalls durch die Steuereinrichtung 6 gesteuerte Ventil A12 gesteuert. Die der Sterilisierkammer 3 zuströmende Luft wird im unteren Bereich der Sterilisierkammer 3 in die Sterilisierkammer 3 eingeleitet. Unmittelbar im Mündungsbereich der Leitungseinrichtung L3 ist hierbei eine Prallplatte und eine Lochblecheinrichtung vorgesehen. Die durch die einzelnen Bohrungen der Lochblecheinrichtung 14 hindurchtretende Druckluft verdrängt das in der Kammer 3 befindliche Dampfmedium und treibt dieses durch die im oberen Bereich der Sterilisierkammer 3 vorgesehene Lochblechanordnung in die Leitungseinrichtung L2. Der aus der Sterilisierkammer 3 verdrängte Dampf und ggf. auch ein Teil der sich in den Dampf einmischenden Druckluft werden über die Leitungseinrichtung L2 und das nach Maßgabe der Steuereinrichtung 6 in eine Offenstellung geschaltete Ventil A7 dem Abluft/Abdampfkanal 4 zugeführt. Dieser Spülvorgang erfolgt weitgehend bei Umgebungsdruck.

Der Zustand des über die Leitungseinrichtung L2 aus der Sterilisierkammer 3 abgeführten Dampf- bzw. Mischmediums wird durch die Sensoren I3 und I6 weiterhin überwacht. Sobald der Dampfanteil in dem über die Leitungseinrichtung L2 abgeführten Medium unter einem vorgegebenen Grenzwert von beispielsweise 5 Gew.-% liegt, werden die Ventile A7 und A12 geschlossen.

Nunmehr setzt die Steuereinrichtung 6 die Pumpe P in Betrieb und saugt die in der Sterilisierkammer 3 befindliche, ggf. durch den Filter F3 gefilterte oder über das Ventil A6 strömende Luft ab. Da die Pumpe P lediglich erwärmte Luft aus der Sterilisierkammer 3 absaugt, ist es nicht erforderlich, die Pumpe P, wie bislang üblich, zu kühlen.

Die in Betrieb gesetzte Pumpe P saugt kontinuierlich über die Leitungseinrichtungen L6, L7 und L2 die vorab in die Kammer 3 eingebrachte erwärmte Luft ab und senkt dabei kontinuierlich den Druck im Inneren der Sterilisierkammer 3. Sobald ein vorgegebener Trocken-Druckpegel, beispielsweise P_{abs} = 20 mbar erreicht ist, wird der in der Kammer eingestellte Unterdruck ggf. unter Verringerung der Drehzahl der Pumpe P auf dem Trocken-Druckpegel gehalten.

Nach Ablauf einer vorbestimmten Trocken-Zeitperiode wird das Ventil A6 geschlossen und die Pumpe P abgeschaltet. Durch Öffnen der Ventile A2, A12 und A17 wird die Sterilisierkammer 3 mit gefilterter und durch die Abwärme des Sterilisierdampfes erwärmter Luft belüftet. Auch die Ventile A1, A3, A9, A11 und A15 werden geschlossen. Sobald durch kontinuierliches Nachströmen der erwärmten Druckluft über die Leitungseinrichtung L3 ein Druckausgleich mit dem Umgebungsdruck erreicht ist, wird die Kammerverriegelung freigegeben, und die Sterilisierkammer 3 wird zur Entnahme des Sterilgutes 1 geöffnet. Die für das gesamte Behandlungsverfahren des Sterilgutes 1 charakteristischen Verfahrensparameter werden von der elektronischen Steuereinrichtung 6 aufgezeichnet und können bedarfsweise später, beispielsweise unter Angabe einer Steriiisier-Chargennummer oder anderweitigen Kennung abgefragt werden. Die Steuereinrichtung ist in vorteilhafter Weise an ein hausseitiges Rechnernetz (LAN) angeschlossen.

Die Erfindung ist nicht auf das vorangehend beschriebene Ausführungsbeispiel beschränkt. Beispielsweise ist es auch möglich, anstelle der über die Leitungseinrichtung L3 zugeführten Druckluft ein beispielsweise durch Mischung von Stickstoff und Kohlendioxyd gebildetes Gasgemisch der Sterilisierkammer 3 zuzuleiten. Das der Sterilisierkammer 3 zugeleitete Gasmedium kann zur Steigerung der Trocknungswirkung im Inneren der Kammer vorab beispielsweise durch eine Salz-Schüttung getrocknet werden. Da bei dem erfindungsgemäßen Verfahren insbesondere das zur Durchführung des Trocknungsschrittes erforderliche Hochvakuum durch eine ungekühlte, beispielsweise als trocken laufende Drehkolbenpumpe ausgebildete Vakuumpumpe erzeugt werden kann, kann auf die bislang verwendete, insbesondere im Hinblick auf die erforderliche Kühlwasserbereitstellung hinsichtlich ihrer Betriebskosten teure Wasser-Ring-Vakuumpumpe verzichtet werden. Die Durchleitung des Sterilisierdampfes durch den Mantel 2 erweist sich insbesondere bei Heißdampf-unverträglichem Sterilgut 1 als besonders vorteilhaft. Bei vergleichsweise unempfindlichem Sterilgut 1 kann jedoch der Sterilisierdampf unmittelbar dem Dampfnetz entnommen werden. Durch die im Rahmen des Trockenschrittes vor Absenken des Druckes in der Kammer durchgeführte Spülung mit erwärmter Luft wird neben einer deutlichen Verringerung der zur Absenkung des Druckes in der Kammer erforderlichen Pumpenleistung auch ein effektiveres Trocknen des Sterilguts erreicht.

## Patentansprüche

1. Dampfsterilisations- bzw. Desinfektionsverfahren zur Behandlung von Sterilgut im Inneren einer Kammer, bei welchem
- im Rahmen eines vorbereitenden, der Gasentfernung dienenden Verfahrensschrittes ein zunächst in der Kammer befindliches Gas-Medium aus dieser weitgehend entfernt wird, indem durch Absaugen des Gas-Mediums der Druck im Inneren der Kammer abgesenkt wird, bevor Dampf zur Bildung eines aus dem verbliebenen Gas-Medium und dem Dampf bestehenden Misch-Mediums in die Kammer eingebracht wird, bis ein bestimmter Überdruckpegel erreicht ist und das gebildete Misch-Medium anschließend aus der Kammer ausgebracht wird,
- im Rahmen eines nachfolgenden Sterilisierschrittes zum Abtöten etwaiger Keime, Dampf in die Kammer eingebracht wird und in der Kammer unter Aufrechterhaltung eines hinsichtlich Druck und/oder Temperatur definierten Dampfzustandes über eine vorbestimmte Zeitperiode verbleibt und
- im Rahmen eines Trocknungsschrittes zum Trocknen des Sterilgutes der Druck aus der Kammer weitgehend entfemt wird, die Kammer nach Angleich des Druckes in der Kammer an den Umgebungsdruck geöffnet wird, und das behandelte Sterilgut aus der Kammer entnommen wird,
' **dadurch gekennzeichnet, daß**
- im Rahmen des Trocknungsschrittes vor einem Absenken des Druckes auf einen Trocken-Druckpegel unterhalb des Umgebungsdruckes für eine vorbestimmte Trokken-Haltezeit ein nicht-kondensierbares Gas-Medium, insbesondere Luft, in die noch mit Dampf gefüllte Kammer eingebracht und dadurch der Dampf weitgehend, aus der Kammer verdrängt wird.

2. Verfahren nach Anspruch 1, **dadurch gekennzeichnet, daß** im Rahmen des Trocknungsschrittes vor Einbringen des nicht-kondensierbaren Gases der Druck im Inneren der Kammer im wesentlichen auf Umgebungsdruck abgesenkt wird.

3. Verfahren nach Anspruch 1 oder 2, **dadurch gekennzeichnet, daß** der im Rahmen des Trocknungsschrittes verdrängte Dampf oder ein mit diesem gebildetes Misch-Medium im Zustand weitgehender Gleichheit zwischen dem Druck im Inneren der Kammer und dem Umgebungsdruck aus der Kammer ausgebracht wird.

4. Verfahren nach einem der Ansprüche 1 bis 3, **dadurch gekennzeichnet, daß** im Rahmen des Trocknungsschrittes zumindest ein Teil des in der Kammer gebildeten Misch-Mediums unter geringem Überdruck aus der Kammer abgelassen, insbesondere ausgespült wird.

5. Verfahren nach Anspruch 3, **dadurch gekennzeichnet, daß** die Kammer mittels des nicht-kondensierbaren Gases gespült wird, bis der Dampfanteil in der Kammer auf einen vorgeschlagenen Wert, vorzugsweise unterhalb 10 Vol.% abgesunken ist

6. Verfahren nach einem der Ansprüche 1 bis 5, **dadurch gekennzeichnet, daß** das im Rahmen des Trocknungsschrittes eingebrachte Gas-Medium derart in die Kammer engebracht wird, daß dieses den in der Kammer noch befindlichen Dampf zu einem Ablaßabschnitt hin verdrängt.

7. Verfahren nach Anspruch 6, **dadurch gekennzeichnet, daß** das im Rahmen des Trocknungsschrittes der Kammer zugeführte Gas-Medium solange zugeführt wird, bis in der Kammer im wesentlichen Umgebungsdruck oder ein höherer Druckpegel herrscht.

8. Verfahren nach einem der Ansprüche 1 bis 7, **dadurch gekennzeichnet, daß** das Gas-Medium im Rahmen des Trocknungsschrittes in die Kammer unter Bildung einer Verdrängungsströmung eingeführt wird.

9. Verfahren nach einem der Ansprüche 1 bis 8, **dadurch gekennzeichnet, daß** das Gas-Medium im Rahmen des Trocknungsschrittes derart zugeführt wird, daß dieses zunächst den unteren Bereich der Kammer erfüllt.

10. Verfahren nach einem der Ansprüche 1 bis 9, **dadurch gekennzeichnet, daß** das verdrängte Medium im Rahmen des Trocknungsschrittes im oberen Bereich der Kammer aus dieser abgeführt wird.

11. Verfahren nach einem der Ansprüche 1 bis 10, **dadurch gekennzeichnet, daß** im Rahmen des Trocknungsschrittes das Gas-Medium an mehreren Orten gerichtet in die Kammer eingeleitet wird, insbesondere über eine Lochblechanordnung derart gleichmäßig gerichtet, die Kammer ausfüllt, daß der Dampf vor einer durch das Gas-Medium gebildeten Gas-Front zu einem Auslaßbereich hin ausgeschoben wird.

12. Verfahren nach einem der Ansprüche 1 bis 11, **dadurch gekennzeichnet, daß** das in der Kammer befindliche Medium über einen großflächig ausgebildeten Austrittsbereich aus der Kammer abgesaugt wird.

13. Verfahren nach einem der Ansprüche 1 bis 12, **dadurch gekennzeichnet, daß** die Absaugung des in der Kammer befindlichen Mediums mittels einer Vakuumpumpe (P) erfolgt.

14. Verfahren nach einem der Ansprüche 1 bis 12, **dadurch gekennzeichnet, daß** das zur Verdrängung des Dampfes im Rahmen des Trocknungsschrittes eingesetzte Gas-Medium vor Eintritt in die Kammer erwärmt bzw. erhitzt und/oder getrocknet wird.

15. Verfahren nach einem der Ansprüche 1 bis 13, **dadurch gekennzeichnet, daß** der Dampfanteil im Inneren der Kammer erfaßt wird.

16. Verfahren nach einem der Ansprüche 1 bis 15, **dadurch gekennzeichnet, daß** der Überdruckpegel in etwa dem halben im Rahmen des Sterilisierschrittes eingestellten Sterilisierdruckpegel entspricht.

17. Verfahren nach einem der Ansprüche 1 bis 16, **dadurch gekennzeichnet, daß** im Rahmen des vorbereitenden Verfahrensschrittes mehrfach Dampf der Kammer zugeführt wird und nach jedem Dampfzufuhrschritt das gebildete Misch-Medium aus der Kammer abgelassen wird.

18. Verfahren nach zumindest einem der Ansprüche 1 bis 17, **dadurch gekennzeichnet, daß** der im Rahmen des vorbereitenden Verfahrensschrittes eingebrachte Dampf derart in die Kammer eingeleitet wird, daß dieser das in der Kammer zunächst befindliche Gasmedium zu einem Ablaßabschnitt hin verdrängt.

19. Verfahren nach zumindest einem der Ansprüche 1 bis 18, **dadurch gekennzeichnet, daß** während des Ablassens des Misch-Mediums weiterhin Dampf in die Kammer nachgeführt wird.

20. Verfahren nach zumindest einem der Ansprüche 1 bis 19, **dadurch gekennzeichnet, daß** der Dampf Sattdampf ist.

21. Vorrichtung zur Durchführung eines Dampfsterilisations- bzw. Desinfektionsverfahrens zur Behandlung von Sterilgut im Inneren einer Kammer, insbesondere nach einem Verfahren gemäß einem der Ansprüche 1 bis 20 mit
- einer Kammer (3) zur Aufnahme von zu behandelndem Sterilgut (1),
- einer ersten Einrichtung (L1) zur Einleitung von Dampf in die Kammer (3), einer zweiten Einrichtung (L2) zur Einleitung eines nicht kondensierbaren Gases, insbesondere Luft in die Kammer (3),
- einer Ableitungseinrichtung (L3) zur Ableitung eines in der Kammer befindlichen Mediums aus der Kammer (3), einer ersten Ventileinrichtung (A1,A15,A3,A4) zur Steuerung der Zuleitung von Dampf nach Maßgabe einer Steuereinheit (6), einer zweiten Ventileinrichtung (A2,A16,A17,A12) zur Steuerung der Zuleitung des nicht-kondensierbaren Gases nach Maßgabe der Steuereinheit (6),
- einer dritten Ventileinrichtung (A2,A6,A7) zur Steuerung des Volumenstroms durch die Ableitungseinrichtung (L3), nach Maßgabe der Steuereinheit (6),
**dadurch gekennzeichnet, daß**
- ein nach dem Sterilisierabschnitt in der Kammer (3) befindliches Dampf-Medium zumindest teilweise über die Ableitungseinrichtung (L3) verdrängbar ist, indem über die zweite Einleitungseinrichtung das nicht kondensierbare Gas-Medium zeitgleich zuleitbar ist, wobei die Steuereinheit (6) die Ventileinrichtungen individuell ansteuert, und daß
- eine Vakuumpumpe (P) vorgesehen ist, mittels der in der Kammer (3) ein Unterdruck durch Absaugen eines in der Kammer (3) befindlichen Mediums erzeugbar ist.

22. Vorrichtung nach Anspruch 21, **dadurch gekennzeichnet, daß** eine Wärmetauschereinrichtung vorgesehen ist, durch die das der Kammer (3) zugeführte, nicht kondensierbare Gas erwärmbar ist.

23. Vorrichtung nach Anspruch 21, **dadurch gekennzeichnet, daß** die Wärmetauschereinrichtung (5) mit der Ableitungseinrichtung verbunden ist, und daß dadurch das über die Zufuhrleitung (10) zugeführte, nicht kondensierbare Gas vor Eintritt in die Kammer (3) durch den abgeführten Dampf erwärmbar ist.

24. Vorrichtung nach einem der Ansprüche 21 bis 23, **dadurch gekennzeichnet, daß** eine Lochblecheinrichtung (14) vorgesehen ist, durch die das nicht kondensierbare Gas in die Kammer (3) großflächig anleitbar ist.

25. Vorrichtung nach einem der Ansprüche 21 bis 24 **dadurch gekennzeichnet, daß** die Vakuumpumpe (P) als trocken laufende Pumpe, insbesondere Drehkolbenpumpe ausgebildet ist.

## Claims

1. Steam sterilisation and/or disinfection process for the treatment of sterile material in the interior of a chamber, in which
- within the scope of a preparatory process step serving for the removal of gas a gaseous medium initially present in the chamber is largely removed from the latter by reducing the pressure in the interior of the chamber by aspirating the gaseous medium, before steam is introduced into the chamber in order to form a mixed medium consisting of the remaining gaseous medium and the steam, until a specific excess pressure level is reached, following which the resultant mixed medium is removed from the chamber
- within the scope of a following sterilisation step in order to kill any possible germs, steam is introduced into the chamber and remains in the chamber for a predetermined duration while maintaining a steam state defined with regard to pressure and/or temperature, and
- within the scope of a drying step for drying the sterile material the pressure in the chamber is largely released, the chamber is opened after equalising the pressure in the chamber to the ambient pressure, and the treated sterile material is removed from the chamber, **characterised in that**
- within the scope of the drying step, before reducing the pressure to a drying pressure level below the ambient pressure for a predetermined drying holding time, a non-condensable gaseous medium, in particular air, is introduced into the chamber still filled with steam and the steam is thereby largely displaced from the chamber.

2. Process according to claim 1, **characterised in that** within the scope of the drying step the pressure in the interior of the chamber is reduced substantially to ambient pressure before introducing the non-condensable gas.

3. Process according to claim 1 or 2, **characterised in that** the steam displaced within the scope of the drying step or a mixed medium formed with the steam is removed from the chamber in a state of substantial equality between the pressure in the interior of the chamber and the ambient pressure.

4. Process according to one of claims 1 to 3, **characterised in that** within the scope of the drying step at least part of the mixed medium formed in the chamber is discharged, in particular flushed out, from the chamber under a slight excess pressure.

5. Process according to claim 3, **characterised in that** the chamber is flushed by means of the non-condensable gas until the proportion of steam in the chamber has fallen to a proposed value, preferably below 10 vol.%.

6. Process according to one of claims 1 to 5, **characterised in that** the gaseous medium introduced within the scope of the drying step is introduced into the chamber in such a way that it displaces the steam still present in the chamber to a discharge section.

7. Process according to claim 6, **characterised in that** the gaseous medium added to the chamber within the scope of the drying step is added until substantially ambient pressure or a higher pressure level prevails in the chamber.

8. Process according to one of claims 1 to 7, **characterised in that** the gaseous medium is introduced within the scope of the drying step into the chamber with the formation of a displacement flow.

9. Process according to one of claims 1 to 8, **characterised in that** the gaseous medium is added within the scope of the drying step in such a way that it initially fills the lower region of the chamber.

10. Process according to one of claims 1 to 9, **characterised in that** within the scope of the drying step the displaced medium present in the upper region of the chamber is removed from the latter.

11. Process according to one of claims 1 to 10, **characterised in that** within the scope of the drying step the gaseous medium is introduced directionally into the chamber at several points, in particular through a perforated metal sheet arrangement uniformly aligned so as to fill the chamber, and that the steam is displaced in front of a gas front formed by the gaseous medium to a discharge region.

12. Process according to one of claims 1 to 11, **characterised in that** the medium present in the chamber is aspirated from the chamber via an outlet region formed over a large area.

13. Process according to one of claims 1 to 12, **characterised in that** the aspiration of the medium present in the chamber is carried out by means of a vacuum pump (P).

14. Process according to one of claims 1 to 12, **characterised in that** the gaseous medium used for the displacement of the steam within the scope of the drying step is warmed or heated and/or dried before entry into the chamber.

15. Process according to one of claims 1 to 13, **characterised in that** the steam fraction in the interior of the chamber is collected.

16. Process according to one of claims 1 to 15, **characterised in that** the excess pressure level corresponds to roughly half the sterilisation pressure level adjusted within the scope of the sterilisation step.

17. Process according to one of claims 1 to 16, **characterised in that** within the scope of the preparatory process step steam is repeatedly added to the chamber and after each steam addition step the mixed medium that is formed is discharged from the chamber.

18. Process according to at least one of claims 1 to 17, **characterised in that** the steam introduced within the scope of the preparatory process step is fed into the chamber in such a way that it displaces the gaseous medium initially present in the chamber to a discharge section.

19. Process according to at least one of claims 1 to 18, **characterised in that** during the discharge of the mixed medium steam is in addition fed into the chamber.

20. Process according to at least one of claims 1 to 19, **characterised in that** the steam is saturated steam.

21. Apparatus for carrying out a steam sterilisation and/or steam disinfection process for the treatment of sterile material in the interior of a chamber, in particular by a process according to one of claims 1 to 20, comprising
- a chamber (3) for receiving sterile material (1) to be treated,
- a first device (L1) for introducing steam into the chamber (3), a second device (L2) for introducing a non-condensable gas, in particular air, into the chamber (3),
- a discharge device (L3) for discharging a medium present in the chamber from the said chamber (3), a first valve arrangement (A1, A15, A3, A4) for controlling the inflow of steam according to a control unit (6), a second valve arrangement (A2, A16, A17, A12) for controlling the inflow of the non-condensable gas according to the control unit (6),
- a third valve arrangement (A2, A6, A7) for controlling the volume flow through the discharge device (L3) according to the control unit (6), **characterised in that**
- a steam medium present in the chamber (3) after the sterilisation section can be displaced at least partially via the discharge device (L3) by simultaneously introducing the non-condensable gaseous medium via the second inflow device, wherein the control unit (6) individually controls the valve arrangements, and that
- a vacuum pump (P) is provided by means of which a reduced pressure can be created in the chamber (3) by aspirating a medium present in the chamber (3).

22. Apparatus according to claim 21, **characterised in that** a heat exchange device is provided by means of which the non-condensable gas fed to the chamber (3) can be heated up.

23. Apparatus according to claim 21, **characterised in that** the heat exchange device (5) is connected to the discharge device, and that the non-condensable gas introduced via the inflow line (10) can thereby be heated up by means of the extracted steam before entering the chamber (3).

24. Apparatus according to one of claims 21 to 23, **characterised in that** a perforated metal plate arrangement (14) is provided through which the non-condensable gas can be introduced over a large area into the chamber (3).

25. Apparatus according to one of claims 21 to 24, **characterised in that** the vacuum pump (P) is designed as a dry-running pump, in particular as a rotary-piston pump.

## Revendications

1. Procédé de stérilisation à la vapeur et de désinfection à la vapeur pour le traitement d'un objet stérile à l'intérieur d'une chambre, dans lequel
- dans le cadre d'une étape préliminaire du procédé servant à expulser le gaz, un milieu de gaz situé en premier lieu dans la chambre est en grande partie expulsé de celle-ci, en abaissant la pression à l'intérieur de la chambre par aspiration du milieu de gaz, avant d'introduire dans la chambre de la vapeur en vue de former un milieu de mélange composé du milieu de gaz restant et de la vapeur, jusqu'à ce qu'on atteigne un niveau de surpression déterminé et que le milieu de mélange formé soit ensuite évacué de la chambre,
- dans le cadre d'une étape de stérilisation suivante visant à tuer les germes éventuels, de la vapeur est introduite dans la chambre et reste dans la chambre pendant une période prédéterminée en maintenant un état de vapeur défini sur le plan de la pression et/ou de la température et
- dans le cadre d'une étape de séchage visant à sécher l'objet de stérilisation, la pression est en grande partie évacuée de la chambre, la chambre est ouverte après ajustement de la pression régnant dans la chambre à la pression ambiante, et l'objet de stérilisation traité est retiré de la chambre,
**caractérisé en ce que**,
- dans le cadre de l'étape de séchage, avant un abaissement de la pression à un niveau de pression de séchage en deçà de la pression ambiante pendant un temps d'arrêt de séchage prédéfini, un milieu de gaz non-condensable, en particulier de l'air, est introduit dans la chambre encore remplie de vapeur et la vapeur est chassée en grande partie de la chambre par ce dernier.

2. Procédé selon la revendication 1, **caractérisé en ce que**, dans le cadre de l'étape de séchage, avant l'introduction du gaz non-condensable, la pression est abaissée à l'intérieur de la chambre essentiellement à la pression ambiante.

3. Procédé selon la revendication 1 ou 2, **caractérisé en ce que** la vapeur chassée dans le cadre de l'étape de séchage, ou un milieu de mélange formé avec ladite vapeur, est évacué(e) de la chambre dans un état proche de l'égalité entre la pression à l'intérieur de la chambre et la pression ambiante.

4. Procédé selon l'une des revendications 1 à 3, **caractérisé en ce que**, dans le cadre de l'étape de séchage, au moins une partie du milieu de mélange formé dans la chambre est vidée, en particulier rincée, de la chambre sous une faible surpression.

5. Procédé selon la revendication 3, **caractérisé en ce que** la chambre est rincée au moyen du gaz non-condensable jusqu'à ce que la part de vapeur dans la chambre soit abaissée à une valeur proposée, de préférence au-dessous de 10 % en volume.

6. Procédé selon l'une des revendications 1 à 5, **caractérisé en ce que** le milieu de gaz introduit dans le cadre de l'étape de séchage est introduit dans la chambre de sorte que celui-ci chasse la vapeur se trouvant encore dans la chambre vers une portion d'évacuation.

7. Procédé selon la revendication 6, **caractérisé en ce que** le milieu de gaz amené jusqu'à la chambre dans le cadre de l'étape de séchage est amené jusqu'à ce qu'il règne essentiellement dans la chambre une pression ambiante ou un niveau de pression supérieur.

8. Procédé selon l'une des revendications 1 à 7, **caractérisé en ce que** le milieu de gaz, dans le cadre de l'étape de séchage, est introduit dans la chambre avec formation d'un courant de chasse.

9. Procédé selon l'une des revendications 1 à 8, **caractérisé en ce que** le milieu de gaz est amené dans le cadre de l'étape de séchage de sorte que celui-ci remplit d'abord la partie inférieure de la chambre.

10. Procédé selon l'une des revendications 1 à 9, **caractérisé en ce que** le milieu chassé, dans le cadre de l'étape de séchage, est évacué de la chambre dans la partie supérieure de celle-ci.

11. Procédé selon l'une des revendications 1 à 10, **caractérisé en ce que**, dans le cadre de l'étape de séchage, le milieu de gaz est introduit dans la chambre en plusieurs endroits de manière dirigée, en particulier via un arrangement de tôle perforée de manière uniformément dirigée, de telle sorte qu'une fois la chambre remplie, la vapeur est repoussée vers une zone d'échappement devant un front de gaz formé par le milieu de gaz.

12. Procédé selon l'une des revendications 1 à 11, **caractérisé en ce que** le milieu qui se trouve dans la chambre est aspiré hors de la chambre par une zone de sortie formée sur une grande surface.

13. Procédé selon l'une des revendications 1 à 12, **caractérisé en ce que** l'aspiration du milieu qui se trouve dans la chambre est effectuée au moyen d'une pompe à faire le vide (P).

14. Procédé selon l'une des revendications 1 à 12, **caractérisé en ce que** le milieu de gaz utilisé pour chasser la vapeur dans le cadre de l'étape de séchage, est chauffé ou réchauffé et/ou séché avant d'entrer dans la chambre.

15. Procédé selon l'une des revendications 1 à 13, **caractérisé en ce que** la part de vapeur est détectée à l'intérieur de la chambre.

16. Procédé selon l'une des revendications 1 à 15, **caractérisé en ce que** le niveau de surpression correspond environ à la moitié du niveau de pression de stérilisation réglé dans le cadre de l'étape de stérilisation.

17. Procédé selon l'une des revendications 1 à 16, **caractérisé en ce que**, dans le cadre de l'étape préliminaire du procédé, de la vapeur est amenée jusqu'à la chambre à plusieurs reprises et que, après chaque étape d'amenée de vapeur, le milieu de mélange formé est vidé de la chambre.

18. Procédé selon au moins l'une des revendications 1 à 17, **caractérisé en ce que** la vapeur introduite dans le cadre de l'étape préliminaire du procédé est introduite dans la chambre de sorte que celle-ci chasse le milieu de gaz qui se trouve en premier lieu dans la chambre vers une portion d'évacuation.

19. Procédé selon au moins l'une des revendications 1 à 18, **caractérisé en ce que**, pendant le vidage du milieu de mélange, de la vapeur continue à être introduite dans la chambre.

20. Procédé selon au moins l'une des revendications 1 à 19, **caractérisé en ce que** la vapeur est de la vapeur saturée.

21. Dispositif pour réaliser un procédé de stérilisation à la vapeur ou de désinfection à la vapeur pour le traitement d'objets stériles à l'intérieur d'une chambre, en particulier suivant un procédé selon l'une des revendications 1 à 20, comprenant :
- une chambre (3) pour recevoir l'objet stérile à traiter (1),
- une première installation (L1) pour introduire de la vapeur dans la chambre (3), une deuxième installation (L2) pour introduire un gaz non-condensable, en particulier de l'air dans la chambre (3),
- une installation d'évacuation (L3) pour évacuer de la chambre (3) un milieu qui se trouve dans la chambre, un premier arrangement de soupapes (A1, A15, A3, A4) pour commander l'amenée de vapeur suivant une unité de commande (6), un deuxième arrangement de soupapes (A2, A16, A17, A12) pour commander l'amenée du gaz non-condensable suivant l'unité de commande (6),
- un troisième arrangement de soupapes (A2, A6, A7) pour commander le débit volumétrique traversant l'installation d'évacuation (L3) suivant l'unité de commande (6), **caractérisé en ce que**,
- un milieu de vapeur qui se trouve dans la chambre (3) après la section de stérilisation peut être chassé au moins en partie par l'installation d'évacuation (L3) **en ce que** le milieu de gaz non-condensable peut être amené simultanément par la deuxième installation d'introduction, l'unité de commande (6) commandant individuellement les arrangements de soupapes, et que
- une pompe à faire le vide (P) est prévue, au moyen de laquelle une dépression peut être créée dans la chambre (3) par aspiration d'un milieu qui se trouve dans la chambre (3).

22. Dispositif selon la revendication 21, **caractérisé en ce qu'**une installation d'échangeur thermique est prévue, qui permet de chauffer le gaz non-condensable amené jusqu'à la chambre (3).

23. Dispositif selon la revendication 21, **caractérisé en ce que** l'installation d'échangeur thermique (5) est reliée à l'installation d'évacuation, et qu'ainsi, le gaz non-condensable amené par la conduite d'amenée (10) peut être réchauffé par la vapeur évacuée avant d'entrer dans la chambre (3).

24. Dispositif selon l'une des revendications 21 à 23, **caractérisé en ce qu'**est prévu un arrangement de tôle perforée (14) par lequel le gaz non-condensable peut être guidé sur une grande surface dans la chambre (3).

25. Dispositif selon l'une des revendications 21 à 24, **caractérisé en ce que** la pompe à faire le vide (P) est formée comme pompe fonctionnant à sec, en particulier comme pompe à piston rotatif.
